(19) Europäisches Patentamt
European Patent Office
Office européen des brevets

(11) **EP 4 390 942 A1**

(12) **EUROPEAN PATENT APPLICATION**

(43) Date of publication:
**26.06.2024 Bulletin 2024/26**

(21) Application number: **22215222.5**

(22) Date of filing: **20.12.2022**

(51) International Patent Classification (IPC):
**G16C 60/00** (2019.01)     **G06N 3/00** (2023.01)
**G16C 20/30** (2019.01)     **G16C 20/70** (2019.01)

(52) Cooperative Patent Classification (CPC):
**G16C 60/00;** G06N 3/0464; G06N 3/08;
G16C 20/30; G16C 20/70

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Borealis AG**
**1020 Vienna (AT)**

(72) Inventors:
• **TORRES, Juan Pablo**
**4021 Linz (AT)**

• **JERABEK, Michael**
**4021 Linz (AT)**
• **KIEHAS, Florian**
**4040 Linz (AT)**
• **REITER, Martin**
**4040 Linz (AT)**

(74) Representative: **Ter Meer Steinmeister & Partner
Patentanwälte mbB
Nymphenburger Straße 4
80335 München (DE)**

(54) **IMAGE-BASED PREDICTION OF DUCTILE-TO-BRITTLE TRANSITION TEMPERATURE OF POLYMER COMPOSITIONS**

(57)     The present invention relates to a computer implemented method for predicting the ductile-to-brittle transition temperature (DBTT) of a test polymer composition based on images. Furthermore, a non-transitory computer readable storage medium is provided for tangibly storing computer program instructions capable of being executed by a processor, the computer program instructions defining the steps of the aforementioned computer implemented method. Furthermore, the invention is directed to the use of fracture surface images and values indicative of the ductile-to-brittle transition temperatures (DBTT) of polymer compositions for training a machine learning algorithm.

Fig. 1

**Description**

**Field of the invention**

The present invention relates to a computer implemented method for predicting the ductile-to-brittle transition temperature (DBTT) of a test polymer composition based on images. Furthermore, a non-transitory computer readable storage medium is provided for tangibly storing computer program instructions capable of being executed by a processor, the computer program instructions defining the steps of the aforementioned computer implemented method. Furthermore, the invention is directed to the use of fracture surface images and values indicative of the ductile-to-brittle transition temperatures (DBTT) of polymer compositions for training a machine learning algorithm.

**Technical background**

Polymers exhibit a transition in their capacity to absorb energy when subjected to impact loads. Under predefined test conditions, there will be a temperature below which a polymer will manifest brittle behavior. In other words, it will break under influence of a rather small amount of energy. Above this temperature, the same polymer will respond in a ductile manner and will be able to absorb a comparatively high amount of energy before failing. On a microscopic level, brittle failure is characterized by a sharp, clean break with little or no molecular displacement. In contrast, ductile failure is accompanied by yielding, *i.e.* onset of plastic deformation in vicinity of the impact position. These phenomena are observed for both polymers and polymer compositions without exceptions.

The temperature at which the above outlined transition from the ductile to the brittle behavior takes place is referred to as the ductile-brittle transition temperature (DBTT). For many applications, the DBTT is a key material parameter. Polymer compositions for various applications, for instance for automotive applications, are often required to have low DBTTs. Additionally, tailoring polymer compositions with respect to their DBTT is especially crucial for engineering applications in which the expected temperature covers a wide range. Up to now, development of polymer compositions for such applications involves extensive experimentation. In order to determine the DBTT, the toughness of the polymer compositions needs to be measured over a wide range of temperatures resulting in a high number of experiments that have to be conducted.

This experimental effort could be reduced if a computer-implemented method would be provided that is able to predict the DBTT (or a value indicative of the DBTT) of a composition on the basis of a few, more readily available experimental data. Such a method for predicting the DBTT would likely also be beneficial because it would reduce expenditures and would allow to accelerate the development of new polymer compositions.

Generally, computer-implemented methods for predicting material properties are known in the prior art.

WO 2021 / 110 690 A1, for example, discloses a computer-implemented method for determining a material property, for example Young's modulus, from a representation of a foam sample, for example an image of a foam sample. To that end, a structural feature, especially a microscopic feature related to a pore, wall, cell, strut or node, is derived from the image of the foam sample in a first step. This structural feature is then provided to a material model such as a physical model or a data-driven model. The data-driven model may be parametrized based on training data and may be capable of correlating the input structural features to output material properties without involving any knowledge of laws of physics. Whereas this might be a viable model for foam samples, it cannot be tranferred to other material classes.

Furthermore, US 10,955,362 B2 describes a computer-implemented method for determining the quality of polymers. To that end, a Raman spectrum of a polymer sample is obtained and polymer properties/features are computed by comparing the chemical and/or structural fingerprint to previously stored properties data using a machine learning tool. The algorithms of the machine learning tool are trained with historical Raman data and polymer properties/features of known samples measured in a laboratory with conventional methods. For a desired polymer property, a data set - including the measured polymer properties/features and the respective Raman spectral data is ingested into a database accessible via an application programming interface, API. The method provides reasonable predictions for polymer properties such as the MFR and the ethylene content. Yet, it is not comparably successful in predicting arbitrary polymer properties. Additionally, a laser has to be used in order to obtain Raman spectra. Hence, generation of the input data is associated with high costs for the mesurement equipment and also poses high demands to ensure safety of the operators.

Thus, in view of the above, no computer implemented method is currently available that would be able to make accurate predictions of the DBTT for polymer compositions. Hence, the abovementioned need to provide such a method persists.

**Summary of the Invention**

The above identified need is satisfied by the computer implemented method according to independent claim 1 and the use of fracture surface images and values indicative of the ductile-to-brittle transition temperatures (DBTT) in machine learning according to claim 15. Advantageous embodiments may be derived from the dependent claims.

The computer implemented method for predicting the ductile-to-brittle transition temperature (DBTT) of a test polymer composition comprises at least three steps. In a first step, a machine learning algorithm is trained with training polymer compositions (hereinafter also "training phase"). This is done by mapping data sets extracted from fracture surface images of the training polymer compositions to values indicative of the DBTT of the training polymer compositions. In this context, a value indicative of the DBTT is either the DBTT itself or a value that has a predetermined relation to the DBTT. In a second step, the trained algorithm is fed, as an input, with corresponding data set(s) extracted from at least one fracture surface image of the test polymer composition. A third step of the method comprises receiving, as an output, a value indicative of the DBTT of the test polymer composition. The second and third step may, therefore, also be referred to as the "prediction phase".

This method proved to be a powerful asset in material research and development. Most notably, the value indicative of the DBTT, which the user receives as an output, is an important material property. It is especially important for polymer compositions, above all for polymer compositions that are used for the manufacture of load-bearing elements. Traditionally, however, the determination of values indicative of the DBTT has been quite time consuming and involved extensive use of specialized measurement equipment. More specifically, impact tests or fracture tests of a test material had to be carried out at a variety of different temperatures in order to derive a value indicative of the DBTT. The inventive method, in contrast to that, is able to deliver these values within seconds or a few minutes based on a data set derived from a single experiment at a constant temperature. Hence, the inventive method is a time-efficient alternative to the conventional experimental approach and it does not rely on excessive use of valuable measurement equipment, material and/or energy resources.

These advantages may be considered the reward for the effort which is invested in the first step, *i.e.* the training of the machine learning algorithm. To ensure that the effort, that is initially invested in the training phase, is outweighed by the advantages in the prediction phase, it is preferred that the method according to the present invention is used in an environment in which new materials are continually developed and characterized in terms of their mechanical behavior. In this context, the countless possibilities to compound different polymers and additives in order to obtain new polymer compositions are an ideal prerequisite for integrating and establishing the computer implemented method as a standard tool.

Polymer composition:

In order to exploit the prediction force of the method, it is preferred that the test polymer composition differs from each of the training polymer compositions in at least one feature, *e.g.* in the content of one or more components and/or in the presence of one or more components. Nevertheless, in one embodiment of the method, each of the training polymer compositions and the test polymer composition is a polyolefin composition, preferably a polyolefin composition which comprises from 50.0 to 100.0 wt.-%, more preferably from 60.0 to 99.8 wt.-%, most preferably from 70.0 to 95.0 wt.-% of a polyolefin. The polyolefin may be selected from a virgin polyolefin or a recycled polyolefin or a blend of those. In general, a blend denotes a mixture of two or more components, wherein at least one of the components is polymeric. Such blends can be prepared by mixing the two or more components according to established mixing procedures.

A virgin polyolefin is a material that is newly produced, in particular from monomeric units such as alpha olefins. The term "virgin" may, furthermore, apply to a material which has not yet completed a first use cycle and/or has not yet been recycled.

In contrast to that, the recycled polyolefin is preferably recovered from waste plastic material derived from post-consumer and/or post-industrial waste. Post-consumer waste refers to objects having completed at least a first use cycle (or life cycle), *i.e.* having already served their first purpose; while industrial waste refers to manufacturing scrap, which does not normally reach a consumer. Further preferably, the recycled polyolefin comprises at least 88 wt.-%, more preferably at least 92 wt.-%, most preferably at least 96 wt.%, of polyolefins.

In terms of analytic methods, virgin polyolefins and recycled polyolefins may be differentiated based on the absence or presence of contaminants, *e.g.* limonene and/or fatty acids and/or paper and/or wood.

To be more specific, each of the training polymer compositions and the test polymer composition may be a polyolefin composition and/or a composition which comprises from 50.0 to 100.0 wt.-%, more preferably from 60.0 to 99.8 wt.-%, most preferably from 70.0 to 95.0 wt.-% of a polyolefin, wherein the polyolefin is, in particular, selected from the group consisting of recycled propylene; recycled polyethylene; virgin polyolefin, *e.g.* virgin polypropylene and/or virgin polyethylene; and blends thereof. Blends may have a weight ratio of polypropylene : polyethylene from 20 : 80 to 80 : 20.

Virgin polyolefins, which may also be contained in the composition that is used as training polymer composition and test polymer composition, preferably comprise at least one homopolymer and/or and least one copolymer. The homopolymer may be a polypropylene homopolymer and/or a polyethylene homopolymer. The copolymer may be selected from the group consisting of copolymers of ethylene and an alpha-olefin comonomer having 3-12 carbon atoms, preferably 3-6 carbon atoms, and copolymers of propylene and an alpha-olefin comonomer having 4-12 carbon atoms, preferably 4-6 carbon atoms.

It should be understood that the polyolefin compositions which are used as training polymer composition and test polymer composition may comprise one or more further component(s) apart from the polyolefin described above. The further component(s) may be contained in an amount of 45 wt.-% or less, preferably in an amount of 41 wt.-% or less, more preferably in an amount of 25 wt.-% or less.

Impact modifier

According to another option, the test polymer composition and/or the training polymer compositions is/are polyolefin compositions as described above, further comprising at least one or more impact modifier, wherein the total content of the impact modifier is preferably from 0.1 to 50 wt.-%, more preferably from 2.0 to 45 wt.-%, based on the total weight of the test polymer composition and/or the training polymer compositions.

The impact modifier may be a heterophasic polypropylene. The total content of the heterophasic polypropylene is preferably from 0.1 to 50 wt.-%, more preferably from 2.0 to 45 wt.-%, based on the total weight of the test polymer composition and/or the training polymer compositions. The term heterophasic polypropylene denotes a propylene-based copolymer (*i.e.* a heterophasic propylene copolymer, HECO) with a crystalline matrix phase, which can be a propylene homopolymer or a random copolymer of propylene and at least one alpha-olefin comonomer, and an elastomeric phase dispersed therein. In case of a random heterophasic propylene copolymer (RAHECO), the crystalline matrix phase is a random copolymer of propylene and at least one alpha-olefin comonomer.

The impact modifier may, alternatively or additionally, be a plastomer and/or an elastomer and/or a copolymer of ethylene and methyl acrylate (PEMA). Suitable elastomers may be ethylene/propylene copolymers with different ethylene/propylene ratio (C2/C3 or C3/C2 elastomers), ethylene/butene copolymers (C2/C4 elastomers), ethylene/octene copolymers (C2/C8 elastomers), grafted ethylene elastomers (such as maleic anhydride grafted ethylene elastomers) or C2/C3 and C2/C4 block copolymers, in particular an ethylene based 1-octene elastomer. Commercially available C2C8 impact modifiers are marketed under the trademarks Engage, Queo, Exact, Tafmer, Infuse, Entira and the like. As maleic anhydride grafted ethylene elastomer commercially available impact modifiers marketed under the trademark name Fusabond may be used.

The impact modifier preferably has an MFR$_2$ (2.16 kg, 190 °C) of 1 to 20 g/10 min, more preferably 1 to 10 g/10 min. The impact modifier further preferably has an MFR$_2$ (2.16 kg, 230 °C) of 2 to 12 g/10 min.

Additive

Alternatively or additionally, the test polymer composition and/or the training polymer compositions is/are polyolefin compositions as described above, further comprising at least one additive. The total content of the additive is preferably from 0.01 to 1 wt.-%, more preferably from 0.05 to 0.5 wt.-%, based on the total weight of the test polymer composition and/or the training polymer compositions. Suitable additives are well known in the art and can, for example, be selected from the group consisting of antioxidants, anti-acids, antiblocking agents, UV protecting agents, nucleating agents and antistatic agents and combinations thereof, with antioxidants being the most preferred in the context of the present invention.

Examples of antioxidants which are commonly used in the art, are sterically hindered phenols (such as CAS No. 6683-19-8, also sold as Irganox 1010 FF™ by BASF, or Irganox 225™ by BASF), phosphorous based antioxidants (such as CAS No. 31570- 04-4, also sold as Hostanox PAR 24 (FF)™ by Clariant, or Irgafos 168 (FF)TM by BASF), sulphur based antioxidants (such as CAS No. 693- 36-7, sold as Irganox PS- 802 FL™ by BASF), nitrogen-based antioxidants (such as 4,4'- bis(1,1' -dimethyl- benzyl)diphenylamine), or antioxidant blends.

Anti-acids (so-called acid scavengers) are also commonly known in the art. Examples are calcium stearates, sodium stearates, zinc stearates, magnesium and zinc oxides, synthetic hydrotalcite (*e.g.* SHT, CAS-No. 11097-59-9), lactates and lactylates, as well as calcium stearate (CAS No. 1592-23-0) and zinc stearate (CAS No. 557-05-1);

Common antiblocking agents are natural silica such as diatomaceous earth (such as CAS No. 60676-86-0 (SuperfFloss™), CAS-No. 60676-86-0 (SuperFloss E™), or CAS-No. 60676-86-0 (Celite 499™)), synthetic silica (such as CAS-No. 7631-86-9, CAS-No. 7631-86-9, CAS-No. 7631-86-9, CAS-No. 7631-86-9, CAS-No. 7631-86-9, CAS-No. 7631-86-9, CAS-No. 112926-00-8, CAS-No. 7631-86-9, or CAS-No. 7631-86-9), silicates (such as aluminum silicate (Kaolin) CAS-no. 1318-74-7, sodium aluminum silicate CAS-No. 1344-00-9, calcined kaolin CAS-No. 92704-41-1, aluminum silicate CAS-No. 1327-36-2, or calcium silicate CAS-No. 1344-95-2), synthetic zeolites (such as sodium calcium aluminosilicate hydrate CAS-No. 1344-01-0, CAS-No. 1344-01-0, or sodium calcium aluminosilicate, hydrate CAS-No. 1344-01-0).

UV protecting agents are, for example, Bis-(2,2,6,6-tetramethyl-4-piperidyl)-sebacate (CAS-No. 52829-07-9, Tinuvin 770); 2-hydroxy-4-octyloxy-benzophenone (CAS-No. 1843-05-6, Chimassorb 81).

Nucleating agents like sodium benzoate (CAS No. 532-32-1); 1,3:2,4-bis(3,4-dimethylbenzylidene)sorbitol (CAS 135861-56-2, Millad 3988) may be used.

Suitable antistatic agents are, for example, glycerol esters (CAS No. 97593-29-8) or ethoxylated amines (CAS No. 71786-60-2 or 61791-31-9) or ethoxylated amides (CAS No. 204-393-1).

It is to be understood that further additives may also be included in the polyolefin composition and that the sum of all ingredients always adds up to 100 wt.-% in each of the embodiments described herein.

Training Phase - Input

With reference to the computer implemented method, it is preferred that the training polymer compositions include compositions differing from each other in the types and/or amounts of the polyolefin(s) and/or the optional further component(s) and/or in their structure, e.g. due to the differing conditions applied in their manufacturing process. In this way, the training polymer compositions are representative of the whole population that is relevant and to which the test polymer composition belongs. Compositions differing from each other in the type and/or amount of impact modifier are, for example, particularly preferred candidates for training polymer compositions. It is believed that compositions with a different type and/or amount of impact modifier cover a broad range of impact strengths and are sufficiently distinct so that the trained machine learning algorithm provides robust and accurate outputs for "unseen" compositions with arbitrary impact strength, fracture strength and/or behavior to impact.

Furthermore, in order to be at least fairly representative of the whole population for which predictions of the DBTT should be made, the training in the first step of the method according to the present invention is preferably carried out with data sets extracted from fracture surface images of at least 15, more preferably at least 30, in particular at least 40 different training polymer compositions and/or the training in the first step of the method is preferably carried out with data sets extracted from at least 50, more preferably at least 100, in particular at least 1000 fracture surface images of training polymer compositions. Below the threshold value of 15 for the minimum number of training polymers, reliability of the prediction and the method suffers. Besides that, it is assumed that the benefit of using data sets extracted from fracture surface images of more than 500, in particular more than 250, different training polymer compositions, will not contribute to a meaningful benefit in the accuracy of the method. Similarly, there is a preferred minimum number of different fracture surface images of the training polymer compositions. The deviation of the recommended minimum number of fracture surface images from the recommended minimum number for the different training polymer compositions is due to the fact that it is preferable to collect fracture surface images of one and the same training polymer composition at different measurement conditions, e.g. different temperatures. For example, the fracture surface images of the training polymer compositions in the first step of the method described above have preferably been obtained by instrumented experimentation in a temperature range from -50 °C to +70 °C.

Technically speaking, each of the fracture surface images of the training polymer compositions and/or at least one fracture surface image of the test polymer composition may be photograph of a specimen(s) obtained by a camera, after a Charpy impact test according to ISO 179-2:2020 or an Izod impact test according to ASTM D256 or ISO 180. The fracture surface images of the training polymer compositions and/or at least one fracture surface image of the test polymer composition may, just as well, be derived from said photograph by at least one manipulation step, wherein the manipulation step is preferably selected from the group consisting of filtering; rescaling; cropping; adjusting brightness, *e.g.* by gamma shifting; adjusting contrast; horizontal flipping; vertical flipping; blurring, *e.g.* by adding noise; and combinations thereof. These manipulation steps may be used to augment the available data basis for the training phase, *i.e.* to generate several fracture surface images from one initially available fracture surface image.

Arrangement of the specimen and/or illumination during record of the photograph mentioned hereinabove is/are preferably the same for each of the fracture surface images of the training polymer compositions and at least one fracture surface image of the test polymer composition. Preferably, a top view photograph, in particular a photograph facing the fracture surface in a perpendicular direction, is taken from the specimen(s) at a constant distance.

It is further preferred that in the first step of the computer implemented method according to the present invention, that is the training step, the data sets extracted from the fracture surface images of the training polymer compositions and/or the data set(s) extracted from the at least one the fracture surface image of the test polymer composition comprise(s) a two-dimensional array of values, preferably a 256x64 matrix of values indicative of a color in a pixel of the fracture surface image(s).

Prediction Phase - Input

It has advantages if the input in the second step of the method (prediction phase) is comparable to the input that has been provided in the first step (training phase). This can be achieved in several ways. For example, the fracture surface image of the test polymer composition is preferably obtained by the same instrumented experimentation, more preferably in the same temperature range from -50 °C to +70 °C as the fracture surface images of the training polymer compositions. More specifically, it may be advantageous if the measurement equipment that has been used to obtain the fractured specimens as well as the corresponding surface images of the fractured specimens is the same and/or unchanged for the test polymer composition and the training polymer composition.

However, and this already implicitly follows from the explanations above, the composition of the test polymer preferably differs from the test polymer compositions. Even the components or the type of components in the test polymer composition are not limited to the components or type of components that are known from the pool of training polymer compositions. If the number and diversity of the training polymer compositions has been appropriately chosen, the trained machine algorithm can successfully be applied to chemically deviating polymer compositions. In this case, the value of the computer implemented method is further increased since test polymer compositions do not have to be analyzed with respect to the kind of components comprised to decide whether they are eligible. Instead, the data set(s) are extracted from at least one fracture surface image of the test polymer composition without further experimentation or analysis and the trained machine algorithm is fed with these data as an input.

Furthermore, in an embodiment, the method according to the present invention comprises training a machine learning algorithm with training polymer compositions by mapping data sets extracted from fracture surface images of the training polymer compositions to values indicative of the DBTT of the training polymer compositions in a first step; feeding the trained algorithm, as an input, with corresponding data set(s) extracted from 1 to 3 fracture surface image(s) of the test polymer composition, preferably 1 or 2 fracture surface image(s) of the test polymer composition in a second step; and receiving, as an output, a value indicative of the DBTT of the test polymer composition in a third step.

Operating the method as described above, namely with only a limited number of data sets representative of the test polymer composition as an input for the prediction phase, is beneficial. In particular, it allows to minimize the experimental effort that is associated with the prediction of the DBTT of a test polymer composition.

Computational Implementation

In a preferred embodiment, the machine learning algorithm is based on a convolutional neural network (CNN). In this case, the data sets extracted from the fracture surface images of the training polymer compositions and the test polymer composition preferably are a two-dimensional array of values. Within the CNN, the two-dimensional array of values in the data set(s) extracted from the fracture surface image(s) of the training polymer compositions and/or test polymer composition may have been subjected to at least one pooling step. In such a pooling step, the output of the network at a certain location is replaced with a statistic of the nearby outputs. For example, if the pooling step is a so-called max pooling step, the maximum output within a rectangular neighborhood is returned. The application of a pooling step imparts a certain degree of invariance to translations of the input, *i.e.* the two-dimensional array of values. In other words, if the input is translated by a small amount, the values of the outputs are less likely to change, once a pooling step has been applied.

In terms of the computational realization, the CNN may be implemented using open-source software. For example, the CNN may be implemented based on a 2D AlexNet which is connected to a fully convolutional neural network regressor using the Pytorch libraries.

Artificial Neural Network

The term "artificial neural network" refers to an algorithm that mimics the operation of the human brain. For processing, it comprises artificial neurons arranged in input layers, output layers and hidden layers. In the hidden layers, each neuron receives input signals from other neurons and uses this input for the further computation. The connections between the neurons have weights, the values of which represent the stored knowledge of the network. Learning is the process of adjusting the weights so that the training data can be reproduced as accurately as possible.

A CNN, as mentioned above as a preferred embodiment for the machine learning algorithm, is a specialized type of an artificial neural network. It performs a mathematical operation called "convolution" in at least one of the layers. In a formula, a convolution may be represented by the following equation (1)

$$ s(t) = (x * w)(t) \quad (1), $$

with the first argument (the function x in the formula above) to the convolution being the input, the second argument (the function w in the formula above) being the kernel and the result (the function *s* in the formula above) being the feature map. In machine learning applications, the input is usually a multidimensional array of data. Likewise, the kernel is a multidimensional array of parameters. Both arrays are adapted by the learning algorithm.

In terms of the present computer implemented invention, a machine learning algorithm based on CNN is preferably used when the data sets extracted from the fracture surface images of the training polymer compositions and the test polymer composition are a two-dimensional array of values, preferably a two-dimensional array of values indicative of a color or grey scale in a pixel of fracture surface images. Such signals have a grid-like structure. In other words, these signals can be considered 2-D grids of measurement values of a sample. In another embodiment, the data sets extracted from the fracture surface images of the training polymer compositions and the test polymer composition are a three-dimensional array of values, preferably a three-dimensional array of values indicative of colors (*e.g.* RGB) in a pixel of fracture surface images. In this case, these signals can be considered 3-D grids of measurement values of a sample. Such grid-like structures are particularly suitable as input for a CNN.

Hyperparameter Optimization and Cross-Validation

An additional step of hyperparameter optimization and/or cross-validation may be included in the method. In this case, training step a) comprises a first training step and a second training step and the method comprises at least one additional step selected from cross validation and hyperparameter optimization, the at least one additional step being preferably performed after the first training step and before the second training step. Such an additional step ensures that the model is not overfitting and does not contain prediction bias.

The hyperparameters are the internal variables of the artificial neural network. When the artificial neural network is a CNN, the hyperparameters may be parameters that determine the network structure (*e.g.* kernel size, kernel type, stride, padding, hidden layers, activation functions) and/or parameters that are related to the training (*e.g.* learning rate). Initial values of the hyperparameters may be estimated using systematic and random searches, or heuristics at the beginning of the training phase. Yet, most of the times, this estimation does not hit the mark and does not result in the best possible output. In other words, in order to find the best combination of hyperparameters, an optimization step may be performed according to the common practice in machine learning. Modules within the computational software that has been chosen for the implementation of the method, may be used to perform the outlined optimization procedure of the hyperparameters.

The cross-validation step may be a single cross-validation step or a *k*-fold cross-validation step. A *k*-fold cross-validation step can be carried out with k preferably being a number from 1 to 20, in particular 1 to 10.

For a single cross-validation step ($k$ = 1), a randomly selected portion of the data sets extracted from the fracture surface images of the training polymer compositions is withheld during the first training step. This withheld portion, which may amount up to 40% of the data sets and is commonly denoted as the "validation set", is preferably fed into the algorithm once the first training step has been completed. The extent of accuracy to which the DBTTs are predicted for the validation set, may be regarded as a measure of the effectiveness of the training phase and the quality of the hyperparameters. Furthermore, the hyperparameters may be adapted. Modules within the computational software that is used may take over the outlined optimization procedure of the hyperparameters. As a result, optimized hyperparameters may be determined by grid-search in a nested cross validation loop. At this point, the second training step, *i.e.* a mapping of data sets extracted from fracture surface images of the training polymer compositions to values indicative of ductile-to-brittle transition temperatures of the training polymer compositions, may be performed based on the optimized hyperparameters.

In a 5-fold cross-validation step ($k$ = 5), the data sets extracted from the fracture surface images of the training polymer compositions are split into 5 partitions of comparable size. Four partitions are used for training, while one is used as a validation set. This process is repeated 5 times, with a different partition used as the validation set each time. For evaluation purposes, the average or median of all folds can be calculated. Such an average or median provides a better prediction, however, a *k*-fold cross-validation also increases training cost in terms of computation time.

In any case, the assignment of the data sets to a test set ensemble and a training set ensemble is a critical step in the optional cross-validation. Cross-validation is only reliable when the assignment is in line with the characteristics of the whole dataset. In other words, data of a particular species or class may not be heavily underrepresented. Hence, it may be advisable to supervise whether the technique that is applied in order to obtain a randomly selected portion of the whole data set, yields acceptable results in a sense that the difference between the ensemble distribution and the population distribution is small.

DBTT

The value(s) indicative of the DBTT may be an absolute temperature, or a value that has a predetermined relation to the DBTT temperature, for example a temperature difference. In the scenario in which the value is an absolute temperature, the value may be the ductile-to-brittle-temperature itself. Otherwise, the value may be value that has a predetermined relation to the ductile-to-brittle transition temperature. Thus, the value indicative of the DBTT may be an indirect measure of the DBTT, for example in the form of a temperature difference according to equation (2) below

$$\Delta T = DBTT - T \qquad (2),$$

wherein T is a reference temperature or a temperature at which the fracture surface image of the test polymer composition has been obtained. This allows predictions to be a measure of the relative difference to a temperature of interest. Moreover, the sign of the difference will indicate whether the deviation is in the direction of increasing temperature or decreasing temperature on the temperature scale.

Controlling Step

The output of the computer implemented method according to the present invention may be used to make a decision on the further fate of the test polymer composition. For example, if the test polymer composition is a candidate composition which is suspected to satisfy the demands of a target application, the value indicative of the DBTT which is output by the present method may bring clarity. The output value may, in particular, be used to determine whether the test polymer composition is taken to the next research stage. More generally, the value indicative of the DBTT may be used for controlling a process in a development of application-tailored polymer composition.

Means for Storage and Execution of the Computer Implemented Method

According to another aspect, a non-transitory computer readable storage medium for tangibly storing computer program instructions capable of being executed by a processor is provided, the computer program instructions defining the steps of the method as described above.
However, the present invention also includes further means for storage and execution of the computer implemented method as described above. For example, the invention extends to methods and apparatuses which perform the method described above, including data processing systems which perform these methods and computer readable media containing instructions which when executed on data processing systems cause the systems to perform these methods.
Non-volatile memory can be a local device coupled directly to the rest of the components in the data processing system. A non-volatile memory that is remote from the system, such as a network storage device coupled to the data processing system through a network interface such as a modem or Ethernet interface, can also be used.
In the present disclosure, some functions and operations are described as being performed by or caused by software code to simplify description. However, such expressions are also used to specify that the functions result from execution of the code/instructions by a processor, such as a microprocessor.
Alternatively, or in combination, the functions and operations as described here can be implemented using special purpose circuitry, with or without software instructions, such as using Application-Specific Integrated Circuit (ASIC) or Field-Programmable Gate Array (FPGA). Embodiments can be implemented using hardwired circuitry without software instructions, or in combination with software instructions. Thus, the techniques are limited neither to any specific combination of hardware circuitry and software, nor to any particular source for the instructions executed by the data processing system.
While one embodiment can be implemented in fully functioning computers and computer systems, various embodiments are capable of being distributed as a computing product in a variety of forms and are capable of being applied regardless of the particular type of machine or computer-readable media used to actually effect the distribution.
At least some aspects disclosed can be embodied, at least in part, in software. That is, the techniques may be carried out in a computer system or other data processing system in response to its processor, such as a microprocessor, executing sequences of instructions contained in a memory, such as ROM, volatile RAM, non-volatile memory, cache or a remote storage device.
Routines executed to implement the embodiments may be implemented as part of an operating system or a specific application, component, program, object, module or sequence of instructions referred to as "computer programs." The computer programs typically include one or more instructions set at various times in various memory and storage devices in a computer, and that, when read and executed by one or more processors in a computer, cause the computer to perform operations necessary to execute elements involving the various aspects.
A machine-readable medium can be used to store software and data which when executed by a data processing system causes the system to perform various methods. The executable software and data may be stored in various places including for example ROM, volatile RAM, non-volatile memory and/or cache. Portions of this software and/or data may be stored in any one of these storage devices. Further, the data and instructions can be obtained from centralized servers or peer to peer networks. Different portions of the data and instructions can be obtained from different centralized servers and/or peer to peer networks at different times and in different communication sessions or in a same communication session. The data and instructions can be obtained in entirety prior to the execution of the applications. Alternatively, portions of the data and instructions can be obtained dynamically, just in time, when needed for execution. Thus, it is not required that the data and instructions be on a machine-readable medium in entirety at a particular instance of time.

Examples of computer-readable media include but are not limited to non-transitory, recordable, and non-recordable type media such as volatile and non-volatile memory devices, Read Only Memory (ROM), Random Access Memory (RAM), flash memory devices, floppy and other removable disks, magnetic disk storage media, optical storage media (e.g., Compact Disk Read-Only Memory (CD ROM), Digital Versatile Disks (DVDs), etc.), among others. The computer-readable media may store the instructions.

The instructions may also be embodied in digital and analog communication links for electrical, optical, acoustic or other forms of propagated signals, such as carrier waves, infrared signals, digital signals, etc. However, propagated signals, such as carrier waves, infrared signals, digital signals, etc. are not tangible machine readable medium and are not configured to store instructions.

In general, a machine-readable medium includes any mechanism that provides (i.e., stores and/or transmits) information in a form accessible by a machine (e.g., a computer, network device, personal digital assistant, manufacturing tool, any device with a set of one or more processors, etc.).

In various embodiments, hardwired circuitry may be used in combination with software instructions to implement the techniques. Thus, the techniques are neither limited to any specific combination of hardware circuitry and software nor to any source for the instructions executed by the data processing system.

Use

According to yet another aspect, the use of fracture surface images and values indicative of the DBTT of polymer compositions for training a machine learning algorithm, preferably for training the machine learning algorithm as described above, is disclosed. Use of fracture surface images is advantageous since these images can easily be obtained and reproduced with standard equipment. Furthermore, no particular safety concerns are associated with the acquisition of the fracture surface images so that no special precautionary measures have to be taken. Furthermore, the value indicative of the ductile-to-brittle transition temperature of a polymer composition is experimentally accessible, for example from a series of experimentally obtained impact curves at different temperatures.

**Brief Description of the Drawings**

Embodiments of the present invention are illustrated by way of example and are not limited by the figures of the accompanying drawings in which identical references represent similar elements.

Fig. 1 contains fracture surface photographs of a specimen subjected to a Charpy impact test at different temperatures;

Fig. 2 shows an exemplary flow diagram illustrating the method steps of the method according to the present invention when carried out with a CNN based machine learning algorithm;

Fig. 3 shows an exemplary flow diagram illustrating a part of the method according to the present invention alongside the main input parameters to define the CNN based machine learning algorithm with an open source software;

Fig. 4 shows an exemplary flow diagram illustrating another part of the method according to the present invention alongside the main input parameters to define the FCN (fully convolutional network) with an open source software;

Fig. 5 visualizes the prediction performance of the method according to the present invention for PE/PP-based test polymer compositions;

Fig. 6 illustrates a cross-validation procedure using a 5-fold training dataset split.

**Detailed Description of the Drawings and Examples**

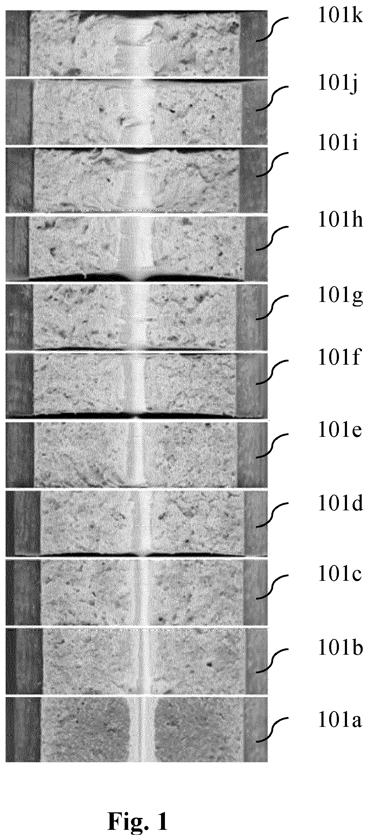

Fig. 1 shows fracture surface photographs 101a-k of eleven specimens. The specimens have been prepared from one and the same polymer composition. The temperature at which the specimens have been subjected to a Charpy impact test according to ISO 179-2:2020 to obtain the fractured surfaces has been increased in 2°C-steps starting from 101a at 0°C to 101k at 20°C.

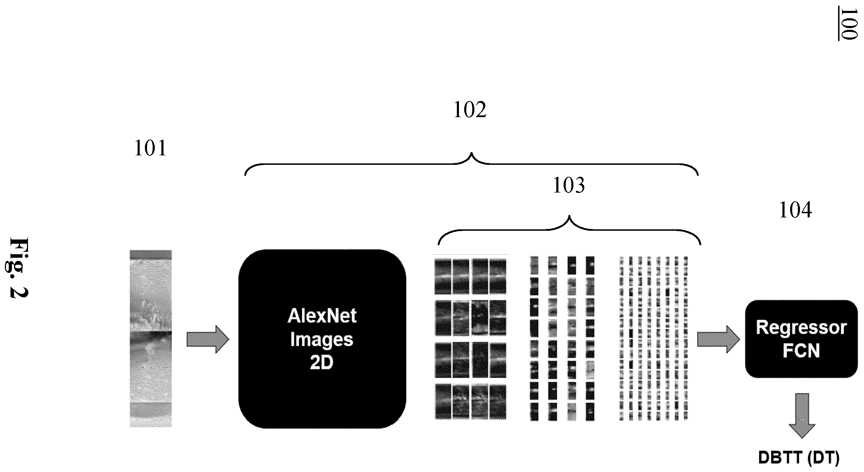

Fig. 2 shows an exemplary flow diagram illustrating the steps of the method according to the present invention when carried out with a CNN based machine learning algorithm.

A dataset which has been derived from an experimentally obtained surface fracture image 101 of a test polymer composition, is fed into a trained CNN based algorithm based on a 2D AlexNet 102.

The CNN performs several convolutions in parallel to produce a set of linear activations. Each linear activation is run through a nonlinear activation function, such as the rectified linear activation function (ReLU). Furthermore, three max pooling operations are used to modify the output of the layer further. A pooling function replaces the output of the net at a certain location with a summary statistic of the nearby outputs. The max pooling operations, illustrated as 103, report the maximum output within a rectangular neighborhood and manage to reduce the size of the dataset that is handled.

Feeding the obtained dataset into the fully convolutional neural network regressor, FCN, 104 allows to obtain the value indicative of the DBTT of the polymer composition.

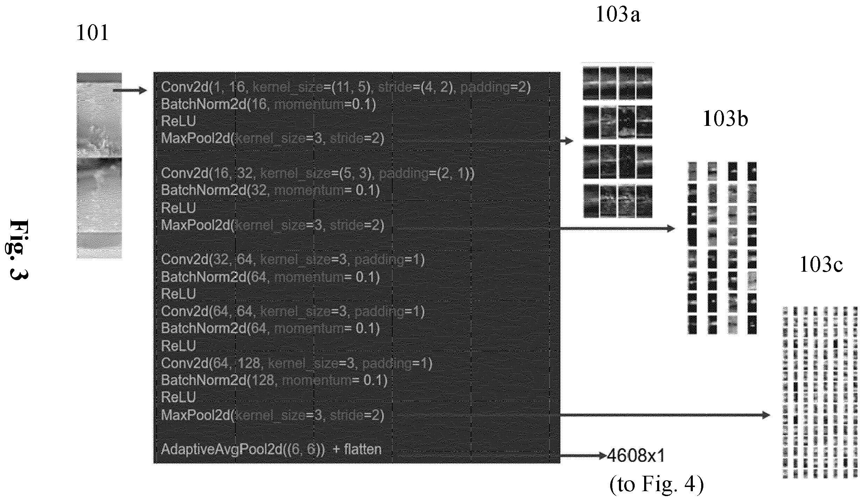

Fig. 3 shows the main input parameters for defining the 2D AlexNet based machine learning algorithm of Fig. 2.

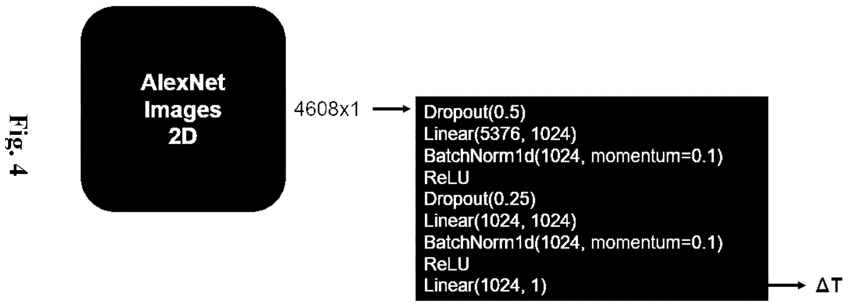

Fig. 4 shows the main input parameters for defining the fully convolutional neural network regressor already shown in Fig. 2.

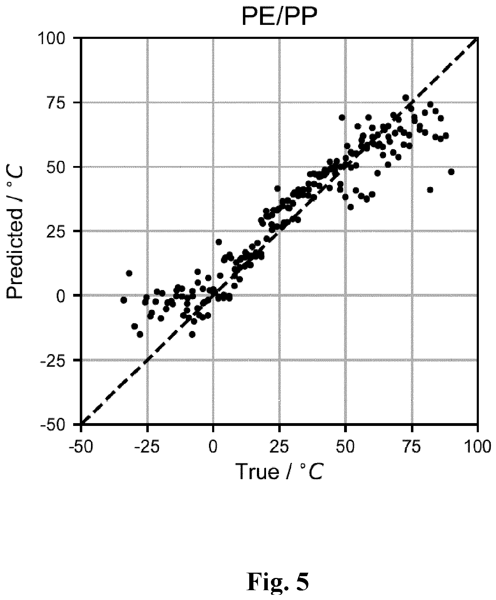

Fig. 5 contains a correlation plot which demonstrates the very good prediction performance of the inventive method for PE/PP-based test polymer compositions. Values indicative of the "true" DBTTs (i.e. experimentally obtained DBTTs) have been plotted on this x-axis whereas the predicted values indicative of the DBTTs have been plotted on the y-axis.

As usual for correlation plots, an ideal prediction would require that all points come to lie exactly on the diagonal line. In the current case, the points in fig. 5 follow the diagonal line fairly well but are slightly scattered to both sides. Yet, the deviation from the diagonal line is, overall, small. Hence, the correlation plot indicates a very good prediction performance of the trained machine learning algorithm.

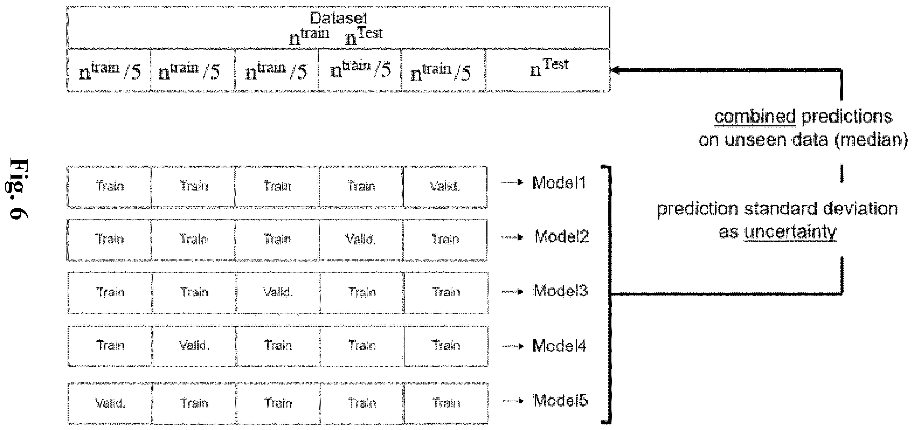

Fig. 6 illustrates a cross-validation procedure which may be adopted in a CNN based machine learning algorithm using five different splits of the training dataset (including 4421 data in total). Five separate trained algorithms are obtained in parallel in this way (Model1-Model5). The median of the outputs of these five models is taken as the final value indicative of the DBTT. Moreover, the standard deviation that may be computed from the outputs of the five models may be considered the uncertainty of the final value indicative of the DBTT.

## Examples

The nature of the present invention will become more clearly apparent in view of the accompanying examples. The examples should, however, in no way limit the scope of the invention.

A CNN based machine learning algorithm based on a 2D AlexNet that is connected to a fully convolutional neural network regressor has been implemented using the Pytorch libraries. It has been trained in the following way:

Training

106 different polymer compositions have been provided. Each of these polymer compositions contained a polyolefin (either PE, PP or both) and an impact modifier. The impact modifier was either a heterophasic propylene copolymer (HECO), an elastomeric or plastomeric impact modified (MOD), or another kind of impact modifier (PO). The polymer compositions have been subjected to an instrumented Charpy test according to according to ISO 179-2:2020 each at a temperature from -40°C to 60°C. Afterwards, top view photographs in a direction perpendicular to the fracture surface of the tested specimen have been taken. This resulted in 5644 fracture surface photographs of broken or notched Charpy specimens. Moreover, a value indicative of the DBTT has been obtained as the "true" value in each experiment.

The polymer compositions have been categorized based on their chemical constitution. Three different chemical categories have been identified: PE/PP (including the subclasses PE_MOD and PE_HECO), PP (including the subclasses PP_MOD and PP_HECO) and PE/PP (including the subclasses PE/PP_MOD, PE/PP_HECO and PE/PP_PO). For each chemical category, a certain number of fracture surface images has been used for the training phase. For PE/PP, for example, data sets extracted from 2957 surface fracture images have been used in training step a).

Furthermore, as can be inferred from fig. 6, the dataset composed of the surface fracture images belonging to 106 different polymer compositions has been divided into a training set, including $n^{train}$ data sets extracted from surface fracture images of training polymer compositions, and a test set, including $n^{Test}$ data sets extracted from surface fracture images of test polymer compositions. Furthermore, the training dataset has been split into 5 folds of roughly equal size ($n^{train}/5$). Balanced distributions of the data to the split portions of the training dataset have been ensured for each of the three chemical categories. In this way, by nested cross-validations, five separate trained algorithms with optimized hyperparameters have been obtained (Model 1-Model5).

After the training phase and cross-validation have been completed, the performance of the trained algorithm has been assessed as outlined below.

Evaluation

The performance of the trained algorithm has been evaluated based on the PE/PP-based test polymer compositions as listed in table 1 below and their surface fracture images. More specifically, data sets extracted from surface fracture images of these test polymer compositions have been input into the trained algorithm in step b) of the method.
Furthermore, table 2 indicates the type of material class that each component belongs to.

Table 1: Details of PE/PP-based polymer compositions.

| Components in compositions |
|---|
| Recycled PE/PP + 20 wt. % Exact8203 |
| Recycled PE/PP + 20 wt. % EBA |
| Recycled PE/PP + 5 wt. % Queo6800LA |
| Recycled PE/PP + 3 wt. % Infuse9077 |

Table 2: Type of material class that each component belongs to.

| Component | Material class |
|---|---|
| Recycled PE/PP | Post-consumer recyclate polyblend PE/PP (commercially available by Borealis AG, with a density according to ISO 1183 of 940 $kg/m^3$ and an $MFR_2$ at 230 °C according to ISO 1133 of 5.5 g/10 min) |
| EBA (commercially available by Borealis) | ethylene butyl acrylate |
| Queo 6800LA (commercially available by Borealis AG) | ethylene based octene-1 elastomer |
| Infuse 9077 (commercially available by Dow) | ethylene octene (C2C8) block copolymer |
| Exact 8203 (commercially available by ExxonMobil Chemicals) | ethylene based octene (C2C8) plastomer |

For each data set extracted from a surface fracture image of a test polymer composition, that has been fed into the trained algorithm, a value indicative of the DBTT has been obtained as an output (*i.e.* the "predicted" value). The output value is the median of the results received from the five separate trained algorithms (Model 1-Model5).
The experimentally obtained true values indicative of the DBTTs of the test polymer compositions have been compared to the predicted values in the correlation plot in Fig. 5. Doing so, the difference between the DBTT and the temperature, at which the fracture surface image of the respective test polymer composition has been obtained, has been assumed to be the value indicative of the DBTT. A very good correlation between true and predicted values has been found.
Furthermore, the performance has also been quantified by numbers: Table 3 below shows the statistics, wherein $n^{train}$ and $n^{Test}$ refer to the number of fracture surface images used during training phase and test phase (*i.e.* prediction phase), respectively. The mean absolute error (MAE) and the coefficient of determination ($R^2$) have been used as scoring functions.

Table 3: Quantified performance of machine learning algorithm for PE/PP-based test polymer compositions.

| | $n^{train}$ | $n^{Test}$ | MAE / °C | $R^2$ |
|---|---|---|---|---|
| **PE/PP** | 2957 | 203 | 8.03 | 0.88 |

**Claims**

1. A computer implemented method for predicting the ductile-to-brittle transition temperature (DBTT) of a test polymer composition, the method comprising the following steps:

   a) training a machine learning algorithm with training polymer compositions by mapping data sets extracted from fracture surface images of the training polymer compositions to values indicative of the ductile-to-brittle transition temperature of the training polymer compositions;
   b) feeding the trained algorithm, as an input, with corresponding data set(s) extracted from at least one fracture surface image of the test polymer composition; and
   c) receiving, as an output, a value indicative of the ductile-to-brittle transition temperature of the test polymer composition.

2. The method of claim 1, wherein each of the training polymer compositions and the test polymer composition is a polyolefin composition, the polyolefin composition preferably comprising from 50 to 100 wt.-%, more preferably from 60.0 to 99.8 wt.-%, most preferably from 70 to 95 wt.-% of a polyolefin, wherein the polyolefin is most preferably selected from the group consisting of recycled or virgin polypropylene, recycled or virgin polyethylene, or a blend thereof.

3. The method of claim 1 or 2, wherein the test polymer composition and/or the training polymer compositions is/are polyolefin compositions comprising one or more impact modifier, wherein the total content of the compatibilizer and/or impact modifier preferably ranges from 0.1 to 50 wt.-%, more preferably from 2.0 to 45 wt.-%, based on the total weight of the test polymer composition and/or the training polymer compositions.

4. The method of one of the preceding claims, wherein the test polymer composition and/or the training polymer compositions is/are polyolefin compositions comprising at least one additive, wherein the total content of the additive is preferably from 0.01 to 1 wt.-%, more preferably from 0.05 to 0.5 wt.-%, based on the total weight of the test polymer composition and/or the training polymer compositions.

5. The method of one of the preceding claims, wherein the training step a) comprises a first training step and a second training step and wherein the method comprises at least one additional step selected from cross validation and hyperparameter optimization, the at least one additional step being preferably performed after the first training step and before the second training step.

6. The method of one of the preceding claims, wherein the training step a) is carried out with data sets extracted from at least 50, preferably at least 100, in particular at least 1000 fracture surface images of training polymer compositions, and/or wherein the training step a) is carried out with data sets extracted from fracture surface images of at least 15, preferably at least 30, in particular at least 40 different training polymer compositions.

7. The method of one of the preceding claims, wherein each of the fracture surface images of the training polymer compositions and the at least one fracture surface image of the test polymer composition have been obtained by instrumented experimentation, preferably in a temperature range from -50 °C to +70 °C.

8. The method of one of the preceding claims, wherein each of the fracture surface images of the training polymer compositions and/or the at least one fracture surface image of the test polymer composition:

   - is/are photograph of specimen(s) obtained by a camera, after a Charpy impact test according to ISO 179-2:2020 or an Izod impact test according to ASTM D256 or ISO180; or
   - is/are derived from said photograph by at least one manipulation step, wherein the manipulation step is preferably selected from the group consisting of filtering, rescaling, cropping, adjusting brightness, adjusting contrast, horizontal flipping, vertical flipping, blurring and combinations thereof.

9. The method of claim 8, wherein camera settings, arrangement of the specimen and/or illumination during record of the photograph is/are the same for each of the fracture surface images of the training polymer compositions and the at least one fracture surface image of the test polymer composition.

10. The method of one of the preceding claims, wherein each of the data sets extracted from the fracture surface images of the training polymer compositions and/or the data set(s) extracted from the at least one the fracture surface image of the test polymer composition comprise(s) a two-dimensional array of values, preferably a 256x64 matrix of values indicative of a color or grey scale in a pixel of the fracture surface image(s).

11. The method of one of the preceding claims, the machine learning algorithm is based on a convolutional neural network, wherein the data set(s) extracted from the fracture surface image(s) of the training polymer compositions and/or the test polymer composition has/have preferably been subjected to a pooling step.

12. The method of one of the preceding claims, wherein the value indicative of the ductile-to-brittle transition temperature is an absolute temperature or a temperature difference.

13. The method of one of the preceding claims, wherein the value indicative of the ductile-to-brittle transition temperature received as the output in step c) is used for controlling a process in a development of an application-tailored polymer composition.

14. A non-transitory computer readable storage medium for tangibly storing computer program instructions capable of being executed by a processor, the computer program instructions defining the steps of one of the preceding method claims.

15. Use of fracture surface images and values indicative of the ductile-to-brittle transition temperature (DBTT) of polymer compositions for training a machine learning algorithm.

101k

101j

101i

101h

101g

101f

101e

101d

101c

101b

101a

**Fig. 1**

Fig. 2

101

103a

103b

103c

Conv2d(1, 16, kernel_size=(11, 5), stride=(4, 2), padding=2)
BatchNorm2d(16, momentum=0.1)
ReLU
MaxPool2d(kernel_size=3, stride=2)

Conv2d(16, 32, kernel_size=(5, 3), padding=(2, 1))
BatchNorm2d(32, momentum= 0.1)
ReLU
MaxPool2d(kernel_size=3, stride=2)

Conv2d(32, 64, kernel_size=3, padding=1)
BatchNorm2d(64, momentum= 0.1)
ReLU
Conv2d(64, 64, kernel_size=3, padding=1)
BatchNorm2d(64, momentum= 0.1)
ReLU
Conv2d(64, 128, kernel_size=3, padding=1)
BatchNorm2d(128, momentum= 0.1)
ReLU
MaxPool2d(kernel_size=3, stride=2)

AdaptiveAvgPool2d((6, 6)) + flatten

4608x1
(to Fig. 4)

Fig. 3

ΔT

Dropout(0.5)
Linear(5376, 1024)
BatchNorm1d(1024, momentum=0.1)
ReLU
Dropout(0.25)
Linear(1024, 1024)
BatchNorm1d(1024, momentum=0.1)
ReLU
Linear(1024, 1)

4608x1

AlexNet
Images
2D

**Fig. 4**

**Fig. 5**

**Fig. 6**

Europäisches
Patentamt

European
Patent Office

Office européen
des brevets

## EUROPEAN SEARCH REPORT

**Application Number**

EP 22 21 5222

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KAISER M. R. ET AL: "Ductile-brittle transition temperature of polylactic acid-based biocomposite", JOURNAL OF THERMOPLASTIC COMPOSITE MATERIALS, vol. 26, no. 2, 2 November 2011 (2011-11-02), pages 216-226, XP093051065, US ISSN: 0892-7057, DOI: 10.1177/0892705711420595 Retrieved from the Internet: URL:http://journals.sagepub.com/doi/full-x ml/10.1177/0892705711420595> * the whole document * ----- | 1-15 | INV. G16C60/00 G06N3/00 ADD. G16C20/30 G16C20/70 |
| A | SUN Y. ET AL: "Predicting Mechanical Properties from Microstructure Images in Fiber-reinforced Polymers using Convolutional Neural Networks", ARXIV.ORG, CORNELL UNIVERSITY LIBRARY, 201 OLIN LIBRARY CORNELL UNIVERSITY ITHACA, NY 14853, 7 October 2020 (2020-10-07), pages 1-10, XP081781243, * the whole document * ----- | 1-15 | |
| A | NAZARI A. ET AL: "Modeling ductile to brittle transition temperature of functionally graded steels by ANFIS", APPLIED MATHEMATICAL MODELLING, vol. 36, no. 8, 20 November 2011 (2011-11-20), pages 3903-3915, XP093050243, GB ISSN: 0307-904X, DOI: 10.1016/j.apm.2011.11.032 * the whole document * ----- -/-- | 1-15 | TECHNICAL FIELDS SEARCHED (IPC) G16C G06N |

The present search report has been drawn up for all claims

| Place of search | Date of completion of the search | Examiner |
|---|---|---|
| The Hague | 2 June 2023 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 2**

Europäisches
Patentamt

European
Patent Office

Office européen
des brevets

**EUROPEAN SEARCH REPORT**

Application Number

EP 22 21 5222

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | STOLL A. ET AL: "Machine learning for material characterization with an application for predicting mechanical properties", GAMM-MITTEILUNGEN, vol. 44, no. 1, 1 March 2021 (2021-03-01), XP055942649, Hoboken, USA ISSN: 0936-7195, DOI: 10.1002/gamm.202100003 Retrieved from the Internet: URL:https://onlinelibrary.wiley.com/doi/full-xml/10.1002/gamm.202100003> * the whole document * | 1-15 | |
| A | ZHANG Y. ET AL: "Analysis of the Effects of Al on the Ductile-to-Brittle Transition Behavior of Ferritic Heat-Resistant Stainless Steels", METALS AND MATERIALS INTERNATIONAL, THE KOREAN INSTITUTE OF METALS AND MATERIALS, SEOUL, vol. 28, no. 7, 24 September 2021 (2021-09-24), pages 1630-1638, XP037892154, ISSN: 1598-9623, DOI: 10.1007/S12540-021-01053-Z [retrieved on 2021-09-24] * the whole document * | 1-15 | TECHNICAL FIELDS SEARCHED (IPC) |

The present search report has been drawn up for all claims

| Place of search | Date of completion of the search | Examiner |
|---|---|---|
| The Hague | 2 June 2023 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021110690 A1
- US 10955362 B2

**Non-patent literature cited in the description**

- *CHEMICAL ABSTRACTS,* 6683-19-8
- *CHEMICAL ABSTRACTS,* 31570- 04-4
- *CHEMICAL ABSTRACTS,* 693- 36-7
- *CHEMICAL ABSTRACTS,* 11097-59-9
- *CHEMICAL ABSTRACTS,* 1592-23-0
- *CHEMICAL ABSTRACTS,* 557-05-1
- *CHEMICAL ABSTRACTS,* 60676-86-0
- *CHEMICAL ABSTRACTS,* 7631-86-9
- *CHEMICAL ABSTRACTS,* 112926-00-8
- *CHEMICAL ABSTRACTS,* 1318-74-7
- *CHEMICAL ABSTRACTS,* 1344-00-9
- *CHEMICAL ABSTRACTS,* 92704-41-1
- *CHEMICAL ABSTRACTS,* 1327-36-2
- *CHEMICAL ABSTRACTS,* 1344-95-2
- *CHEMICAL ABSTRACTS,* 1344-01-0
- *CHEMICAL ABSTRACTS,* 52829-07-9
- *CHEMICAL ABSTRACTS,* 1843-05-6
- *CHEMICAL ABSTRACTS,* 532-32-1
- *CHEMICAL ABSTRACTS,* 135861-56-2
- *CHEMICAL ABSTRACTS,* 97593-29-8
- *CHEMICAL ABSTRACTS,* 71786-60-2
- *CHEMICAL ABSTRACTS,* 204-393-1